# EUROPEAN PATENT APPLICATION

(11) **EP 4 502 871 A1**
(43) Date of publication of application: **05.02.2025**
(21) Application number: 22935544.1
(22) Date of filing: 31.03.2022
(51) Int. Cl.: G06N 5/04

(54) **INFORMATION PROCESSING PROGRAM, INFORMATION PROCESSING DEVICE, AND INFORMATION PROCESSING METHOD**

(71) Applicant: FUJITSU LIMITED, Kawasaki-shi, Kanagawa 211-8588 (JP)
(72) Inventor: KOYANAGI, Yusuke, Kawasaki-shi, Kanagawa 211-8588 (JP); ASAI, Tatsuya, Kawasaki-shi, Kanagawa 211-8588 (JP); MARUHASHI, Koji, Kawasaki-shi, Kanagawa 211-8588 (JP)
(74) Representative: Hoffmann Eitle
(86) International application number: PCT/JP2022/016927
(87) International publication number: WO 2023/188406

(57) **Abstract**

An information processing device (1) receives a hypothesis to be interpreted, uses a first storage unit that includes, for each piece of knowledge that indicates a plurality of resources and a relationship between the resources, basis information that serves as a basis of the knowledge, and a rule identifier connected with a rule used to interpret the hypothesis, to acquire the basis information and the rule identifier that correspond to the hypothesis to be interpreted, and uses a second storage unit that includes, for each rule identifier, a probability that the rule and the hypothesis coincide with existing knowledge, to acquire the probability of coinciding with the existing knowledge that corresponds to the acquired rule identifier. This may enable to interpret a hypothesis, using existing knowledge, in order to sort out the hypothesis.

## Description

### TECHNICAL FIELD

The present invention relates to an information processing program and the like.

### BACKGROUND ART

In recent years, a statistically obtained hypothesis is utilized in hypothesis verification for making a discovery. For example, by focusing on genomic medicine or the like, a causal relationship between gene expressions and a hypothesis in which a causal relationship between a gene and drug resistance has not been clarified are verified, and a discovery is made.

Here, a platform for inputting data and extracting a result (hypothesis) of searching for a statistical causal relationship is disclosed (see, for example, Non-Patent Document). It is desired to sort out the extracted results (hypotheses) by comparison with existing knowledge, or the like.

In order to sort out the hypotheses, an approach of interpreting the extracted results (hypotheses), using existing knowledge is conceivable. For example, in such an approach, whether a hypothesis as an extracted result exists in knowledge data indicating existing knowledge can be confirmed, and the hypothesis can be interpreted based on a result of the confirmation.

### CITATION LIST

### NON-PATENT DOCUMENT

Non-Patent Document 1: S. Budd et al., "Prototyping CRISP: A Causal Relation and Inference Search Platform applied to Colorectal Cancer Data," 2021 IEEE 3rd Global Conference on Life Sciences and Technologies (LifeTech), 2021, pp. 517-521, doi: 10.1109/LifeTech52111.2021.9391819.

### SUMMARY OF INVENTION

### TECHNICAL PROBLEM

However, there is a difficulty that a hypothesis may not be sufficiently interpreted using existing knowledge. For example, in the approach of interpreting a hypothesis using existing knowledge, whether a hypothesis coincides with the existing knowledge or does not coincide with the existing knowledge can be interpreted, but interpretation is the same for hypotheses that do not coincide with the existing knowledge, and a hypothesis that does not coincide with the existing knowledge may not be sufficiently interpreted.

In one aspect, an object of the present invention is to interpret a hypothesis, using existing knowledge, in order to sort out hypothesis.

### SOLUTION TO PROBLEM

In one form, an information processing program causes a computer to execute a process including using a first storage unit that includes, for each piece of knowledge that indicates a plurality of resources and a relationship between the resources, basis information that serves as a basis of the knowledge, and a rule identifier connected with a rule used to interpret a hypothesis to be interpreted, to acquire the basis information and the rule identifier that correspond to the hypothesis; and using a second storage unit that includes, for each rule identifier, a probability that the rule and the hypothesis coincide with existing knowledge, to acquire the probability of coinciding with the existing knowledge that corresponds to the acquired rule identifier.

### EFFECTS OF INVENTION

According to one exemplary form, a hypothesis can be interpreted using existing knowledge in order to sort out hypothesis.

### BRIEF DESCRIPTION OF DRAWINGS

FIG. 1 is a block diagram illustrating an example of a functional configuration of an information processing device according to a first embodiment.
FIG. 2 is a diagram explaining hypothesis interpretation according to the first embodiment.
FIG. 3 is a diagram illustrating an example of a hypothesis interpretation-purpose database (DB) according to the first embodiment.
FIG. 4 is a diagram illustrating an example of interpretation rules according to the first embodiment.
FIG. 5 is a diagram illustrating an example of query information according to the first embodiment.
FIG. 6A is a diagram (1) illustrating an example of hypothesis interpretation according to the first embodiment.
FIG. 6B is a diagram (2) illustrating an example of hypothesis interpretation according to the first embodiment.
FIG. 6C is a diagram (3) illustrating an example of hypothesis interpretation according to the first embodiment.
FIG. 6D is a diagram (4) illustrating an example of hypothesis interpretation according to the first embodiment.
FIG. 7 is a diagram illustrating an example of a flowchart of a hypothesis interpretation process according to the first embodiment.
FIG. 8 is a diagram illustrating an effect according to the first embodiment.
FIG. 9 is a block diagram illustrating an example of a functional configuration of an information processing device according to a second embodiment.
FIG. 10 is a diagram illustrating an example of knowledge data according to the second embodiment.
FIG. 11 is a diagram illustrating an example of interpretation rules according to the second embodiment.
FIG. 12 is a diagram illustrating an example of DB generation according to the second embodiment.
FIG. 13 is a diagram illustrating an example of a flowchart of a DB generation process according to the second embodiment.
FIG. 14 is a block diagram illustrating an example of a functional configuration of an information processing device according to a third embodiment.
FIG. 15A is a diagram (1) illustrating an example of hypothesis interpretation according to the third embodiment.
FIG. 15B is a diagram (2) illustrating an example of hypothesis interpretation according to the third embodiment.
FIG. 15C is a diagram (3) illustrating an example of hypothesis interpretation according to the third embodiment.
FIG. 16A is a diagram (1) illustrating an example of a flowchart of a hypothesis interpretation process according to the third embodiment.
FIG. 16B is a diagram (2) illustrating an example of a flowchart of the hypothesis interpretation process according to the third embodiment.
FIG. 17 is a block diagram illustrating an example of a functional configuration of an information processing device according to a fourth embodiment.
FIG. 18 is a block diagram illustrating an example of a functional configuration of an information processing device according to a fifth embodiment.
FIG. 19 is a diagram illustrating an example of interpretation rule generation according to the fifth embodiment.
FIG. 20 is a diagram illustrating an example of a flowchart of an interpretation rule generation process according to the fifth embodiment.
FIG. 21 is a block diagram illustrating an example of a functional configuration of an information processing device according to a sixth embodiment.
FIG. 22 is a diagram illustrating an example of a hypothesis interpretation DB according to the sixth embodiment.
FIG. 23 is a diagram illustrating an example of a screen image.
FIG. 24 is a diagram illustrating another example of a screen image.
FIG. 25 is a diagram illustrating an example of a computer that executes an information processing program.

### DESCRIPTION OF EMBODIMENTS

Hereinafter, embodiments of an information processing program, an information processing device, and an information processing method disclosed in the present application will be described in detail with reference to the drawings. Note that the present invention is not limited by the embodiments.

### [First Embodiment]

### [Functional Configuration of Information Processing Device]

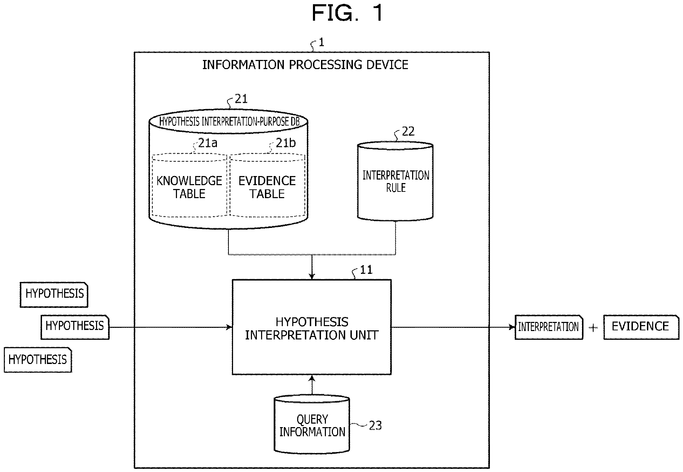

FIG. 1 is a block diagram illustrating an example of a functional configuration of an information processing device according to a first embodiment. The information processing device 1 illustrated in FIG. 1 presents interpretation of knowledge with respect to a hypothesis, using knowledge data and an interpretation rule. The knowledge data mentioned here refers to data that holds each piece of knowledge and basis information (evidence) serving as a basis of each piece of knowledge. The interpretation rule mentioned here refers to a rule for interpreting a hypothesis.

The information processing device 1 includes a hypothesis interpretation unit 11, a hypothesis interpretation-purpose DB 21, an interpretation rule 22, and query information 23. Note that the hypothesis interpretation-purpose DB 21 is an example of a first storage unit. The interpretation rule 22 is an example of a second storage unit.

The hypothesis interpretation-purpose DB 21 is a DB used to interpret a hypothesis. The hypothesis interpretation-purpose DB 21 includes a knowledge table 21a and an evidence table 21b.

The knowledge table 21a stores knowledge according to the structure of the knowledge. The structure of the knowledge mentioned here is, for example, a structure having one resource, another resource, and a relationship between the one resource and the another resource, but is not limited thereto and conforms to the structure of the knowledge of a knowledge base. However, in the following embodiments, it is assumed that the structure having one resource, another resource, and a relationship between the one resource and the another resource is represented as one piece of knowledge, and the structure of a hypothesis has a similar structure.

The evidence table 21b includes, for knowledge, the basis information (evidence) serving as a basis of the knowledge and a rule identifier connected with a rule used to interpret a hypothesis. That is, the evidence table 21b holds evidence of each piece of knowledge. The evidence mentioned here refers to information for identifying papers, documents, and DBs serving as proofs. Note that an example of the hypothesis interpretation-purpose DB 21 will be described later.

The interpretation rule 22 includes, for each rule identifier, a rule used to interpret a hypothesis and a degree of probability of coinciding with existing knowledge. Note that an example of the interpretation rule 22 will be described later.

The query information 23 is a template of a query used when interpreting a hypothesis. Note that an example of the query information 23 will be described later.

The hypothesis interpretation unit 11 interprets a hypothesis, using existing knowledge.

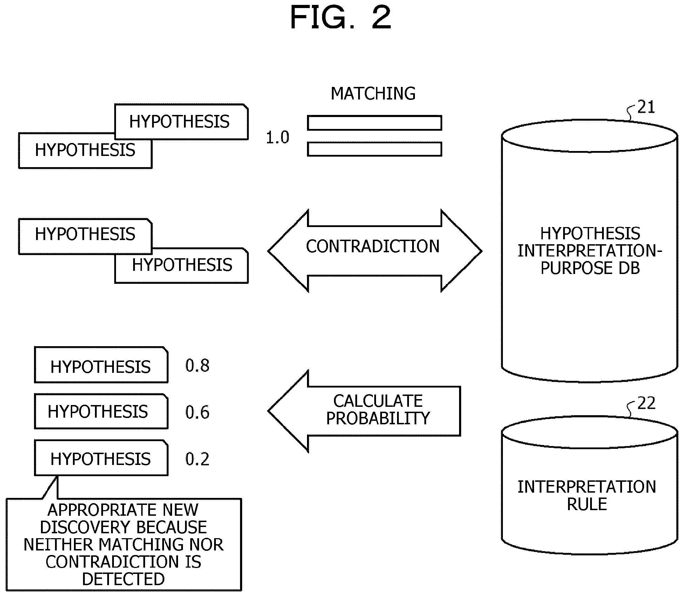

Here, hypothesis interpretation according to the first embodiment will be described with reference to FIG. 2. FIG. 2 is a diagram explaining hypothesis interpretation according to the first embodiment. As illustrated in FIG. 2, the hypothesis interpretation unit 11 uses the hypothesis interpretation-purpose DB 21 and the interpretation rule 22 to interpret whether the hypothesis matches with or is contradictory to existing knowledge, or is a new discovery. At this time, the hypothesis interpretation unit 11 calculates a degree of probability of coinciding with existing knowledge and presents the calculated degree of probability as an interpretation result. For example, the degree of probability of coinciding with existing knowledge means to match with the existing knowledge in a case of "1.0", and a smaller numerical value means a higher possibility of being a new discovery.

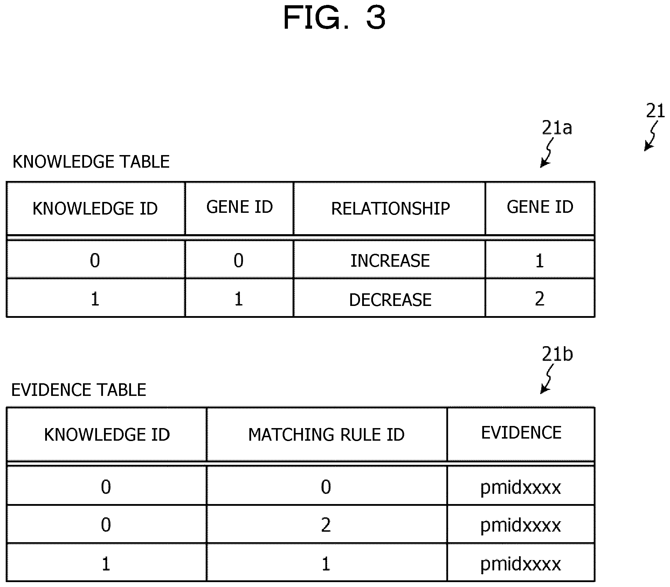

Here, an example of the hypothesis interpretation-purpose DB 21 will be described with reference to FIG. 3. FIG. 3 is a diagram illustrating an example of the hypothesis interpretation-purpose DB according to the first embodiment. The hypothesis interpretation-purpose DB 21 includes the knowledge table 21a and the evidence table 21b.

The knowledge table 21a stores gene identifiers (IDs), relationships, and gene IDs in association with knowledge identifiers (IDs). The gene ID, the relationship, and the gene ID constitute one piece of knowledge. As an example, in a case where the knowledge ID is "0", "0" is stored as one gene ID, "1" is stored as another gene ID, and "increase" is stored as the relationship. That is, the knowledge indicating the relationship in which, when the gene indicated by the one gene ID "0" increases, the gene indicated by the another gene ID "1" also increases is stored.

The evidence table 21b stores evidence in association with the knowledge ID and a matching rule ID. The evidence stores an identifier for identifying a paper, a document, or a DB serving as a proof, for the knowledge. The knowledge ID corresponds to the knowledge ID in the knowledge table 21a. The matching rule ID corresponds to a rule ID in the interpretation rule 22 to be described later. As an example, in a case where the knowledge ID is "0" and the matching rule ID is "0", "pmidxxxx" is stored as evidence.

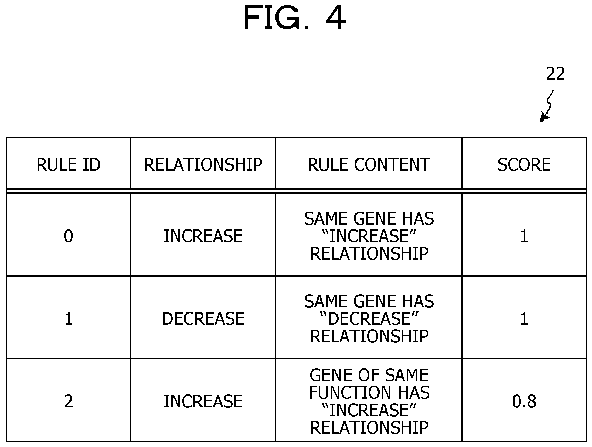

Here, an example of the interpretation rule 22 will be described with reference to FIG. 4. FIG. 4 is a diagram illustrating an example of the interpretation rules according to the first embodiment.

The interpretation rule 22 stores a relationship, rule content, and a score in association with the rule ID. The relationship corresponds to the relationship between resources. The rule content indicates the content of the rule represented by the resources and the relationship. The score indicates a degree of probability of coinciding with existing knowledge. Put in different terms, the score is a degree of matching with existing knowledge. A larger score value means a higher possibility of coinciding with existing knowledge. In addition, a smaller score value means a higher possibility of being an appropriate new discovery. The score is represented by a numerical value between zero and one as an example.

As an example, in a case where the rule ID is "0", "increase" is stored as the relationship, "same gene has "increase" relationship" is stored as the rule content, and "1" is stored as the score. In a case where the rule ID is "2", "increase" is stored as the relationship, "gene of same function has "increase" relationship" is stored as the rule content, and "0.8" is stored as the score.

Returning to FIG. 1, for example, after acquiring a hypothesis to be interpreted, the hypothesis interpretation unit 11 acquires the knowledge ID corresponding to the hypothesis from the knowledge table 21a of the hypothesis interpretation-purpose DB 21. Then, the hypothesis interpretation unit 11 acquires evidence and a list of rules connected with the acquired knowledge ID from the evidence table 21b of the hypothesis interpretation-purpose DB 21 and the interpretation rule 22. Then, the hypothesis interpretation unit 11 acquires the score of the corresponding rule from the acquired list of rules. Then, the hypothesis interpretation unit 11 holds information in which the knowledge ID, the evidence, the matching rule ID, and the score are associated, as a matching result list. Note that the hypothesis interpretation unit 11 may use a query to execute the process from acquiring the hypothesis to be interpreted to holding the matching result list. An example of the query will be described later.

Then, the hypothesis interpretation unit 11 aggregates the held matching result list. Note that, as an example, the hypothesis interpretation unit 11 can simply prioritize information having a higher score in information included in the matching result list at the time of aggregation, but as another example, all the evidence may be written together with the scores as an average value, or the aggregation is not limited thereto.

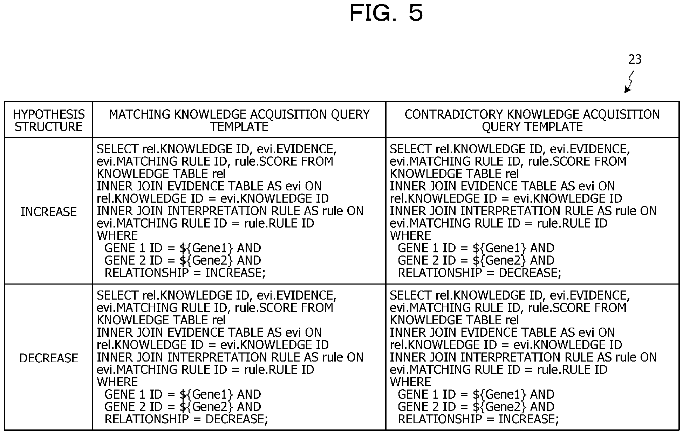

Here, an example of the query information will be described with reference to FIG. 5. FIG. 5 is a diagram illustrating an example of the query information according to the first embodiment. As illustrated in FIG. 5, the query information 23 is information in which a hypothesis structure, a matching knowledge acquisition query template, and a contradictory knowledge acquisition query template are associated. The hypothesis structure corresponds to the relationship between resources indicated by the hypothesis. The matching knowledge acquisition query template is a template of a query for acquiring knowledge matching the hypothesis structure. The contradictory knowledge acquisition query template is a template of a query for acquiring contradictory knowledge that does not match with the hypothesis structure.

As an example, in a case where the hypothesis structure is "increase", "SELECT.... relationship = increase" is stored as the matching knowledge acquisition query template, and "SELECT.... relationship = decrease" is stored as the contradictory knowledge acquisition query template.

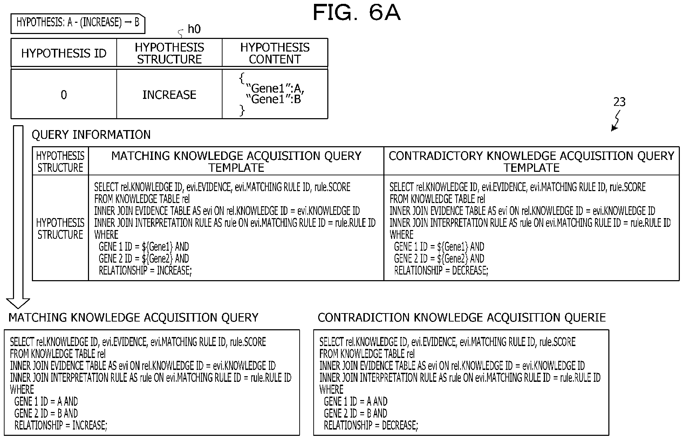

FIGs. 6A to 6D are diagrams illustrating an example of hypothesis interpretation according to the first embodiment. As illustrated in FIG. 6A, it is assumed that hypothesis interpretation unit 11 acquires "A - (increase) → B" (a relationship that B increases as A increases) as a hypothesis to be interpreted. As an example, the hypothesis has the content indicated by the reference sign h0, which is a case where the hypothesis structure is "increase".

Then, the hypothesis interpretation unit 11 acquires the matching knowledge acquisition query template and the contradictory knowledge acquisition query template corresponding to the hypothesis structure of the hypothesis h0 acquired from the query information 23. Here, the matching knowledge acquisition query template and the contradictory knowledge acquisition query template of which the hypothesis structure is "increase" are acquired from the query information 23.

Then, the hypothesis interpretation unit 11 generates a matching knowledge acquisition query and a contradictory knowledge acquisition query from the hypothesis h0, the matching knowledge acquisition query template, and the contradictory knowledge acquisition query template. Here, the matching knowledge acquisition query and the contradictory knowledge acquisition query are generated by substituting "A" and "B" into the "$(Gene1)" and "$(Gene2)" portions of each of the matching knowledge acquisition query template and the contradictory knowledge acquisition query template. The left diagram at the lowermost part of FIG. 6A is the generated matching knowledge acquisition query, and the right diagram at the lowermost part of FIG. 6A is the generated contradictory knowledge acquisition query.

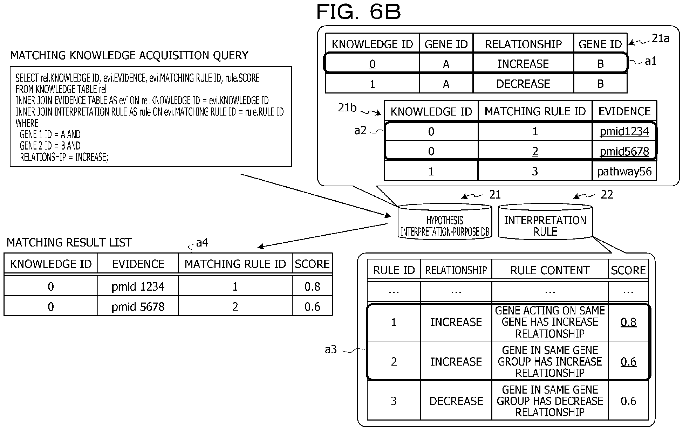

Then, as illustrated in FIG. 6B, the hypothesis interpretation unit 11 inquires of the hypothesis interpretation-purpose DB 21 and the interpretation rule 22, using the matching knowledge acquisition query. The hypothesis interpretation unit 11 acquires a matching result list. Here, "0" is acquired as the knowledge ID (reference sign a1) of which the gene ID is "A", the gene ID is "B", and the relationship is "increase", from the knowledge table 21a. From the evidence table 21b, "1" as one matching rule ID and "pmid1234" as evidence, of which the knowledge ID is "0", are acquired, and "2" as another matching rule ID and "pmid5678" as evidence, of which the knowledge ID is "0", are acquired (reference sign a2). Additionally, from the interpretation rule 22, "0.8" is acquired as the score of which the matching rule ID is "1", and "0.6" is acquired as the score of which the matching rule ID is "2" (reference sign a3). Then, the hypothesis interpretation unit 11 holds information in which the knowledge ID, the evidence, the matching rule ID, and the score are associated, as a matching result list. Here, the matching result list is information indicated by the reference sign a4.

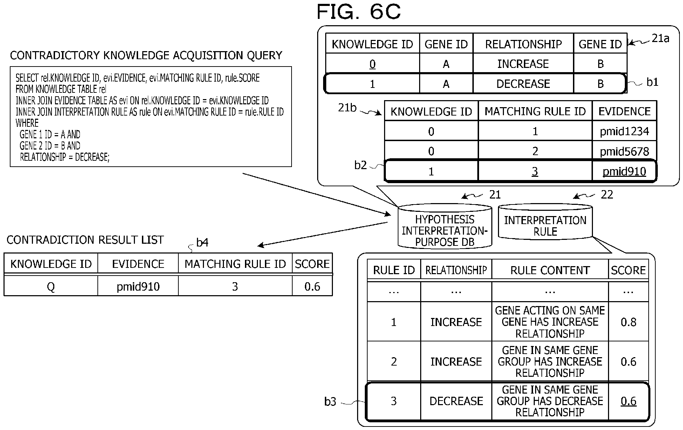

Then, as illustrated in FIG. 6C, the hypothesis interpretation unit 11 inquires of the hypothesis interpretation-purpose DB 21 and the interpretation rule 22, using the contradictory knowledge acquisition query. The hypothesis interpretation unit 11 acquires a contradiction result list. Here, "1" is acquired as the knowledge ID (reference sign b1) of which the gene ID is "A", the gene ID is "B", and the relationship is "decrease", from the knowledge table 21a. From the evidence table 21b, "3" as the matching rule ID and "pmid910" as evidence, of which the knowledge ID is "1", are acquired (reference sign b2). Additionally, from the interpretation rule 22, "0.6" is acquired as the score of which the matching rule ID is "3" (reference sign b3). Then, the hypothesis interpretation unit 11 holds information in which the knowledge ID, the evidence, the matching rule ID, and the score are associated, as a contradiction result list. Here, the contradiction result list is information indicated by the reference sign b4.

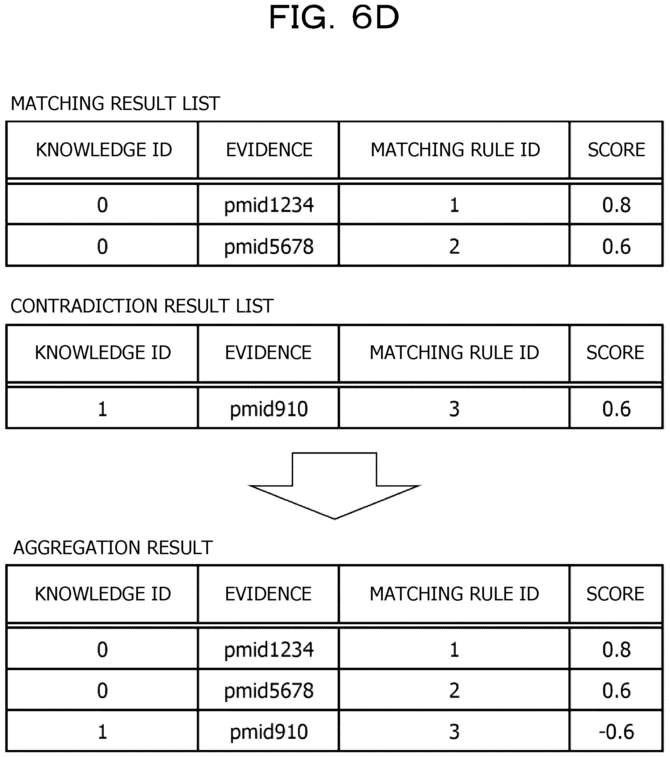

Then, as illustrated in FIG. 6D, the hypothesis interpretation unit 11 aggregates the held matching result list and contradiction result list and holds the aggregated matching result list and contradiction result list in an aggregation result. Here, the aggregation result is held in the order of the matching result list and the contradiction result list in the descending order of scores. Here, the aggregation result of the knowledge ID, the evidence, and the matching rule ID is held in the order of scores of "0.8", "0.6", and "-0.6". The last score of the aggregation result is negative because it is a score for contradictory knowledge.

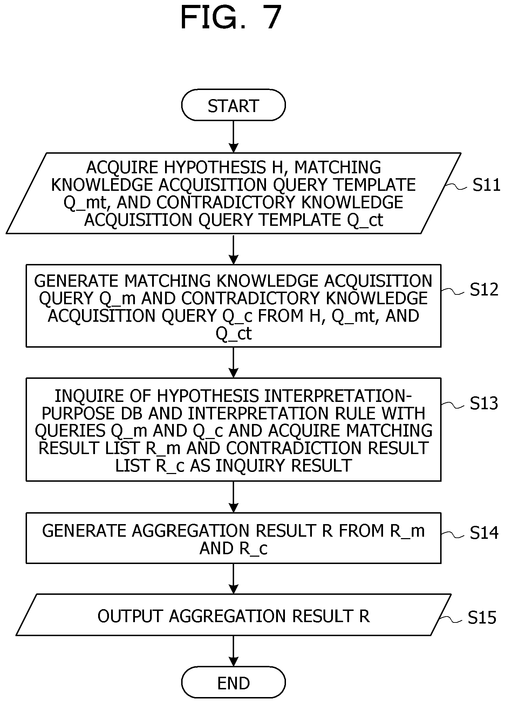

FIG. 7 is a diagram illustrating an example of a flowchart of a hypothesis interpretation process according to the first embodiment.

As illustrated in FIG. 7, the hypothesis interpretation unit 11 acquires a hypothesis H, a matching knowledge acquisition query template Q_mt, and a contradictory knowledge acquisition query template Q_ct (step S11). For example, after acquiring the hypothesis H to be interpreted, the hypothesis interpretation unit 11 acquires, from the query information 23, the matching knowledge acquisition query template Q_mt and the contradictory knowledge acquisition query template Q_ct corresponding to the relationship included in the hypothesis H.

Then, the hypothesis interpretation unit 11 generates a matching knowledge acquisition query Q_m and a contradictory knowledge acquisition query Q_c from the hypothesis H, the matching knowledge acquisition query template Q_mt, and the contradictory knowledge acquisition query template Q_ct (step S12). For example, the hypothesis interpretation unit 11 generates the matching knowledge acquisition query Q m and the contradictory knowledge acquisition query Q_c by substituting a gene IDa and a gene IDb included in the hypothesis H into the matching knowledge acquisition query template Q_mt and the contradictory knowledge acquisition query template Q_ct.

Then, the hypothesis interpretation unit 11 inquires of the hypothesis interpretation-purpose DB (21) and the interpretation rule (22) with the matching knowledge acquisition query Qm and the contradictory knowledge acquisition query Q_c and acquires a matching result list R m and a contradiction result list R c as an inquiry result (step S13). Then, the hypothesis interpretation unit 11 generates an aggregation result R from the matching result list R m and the contradiction result list R_c (step S14).

Then, the hypothesis interpretation unit 11 outputs the aggregation result R (step S15). The hypothesis interpretation unit 11 then ends.

### [Effects of First Embodiment]

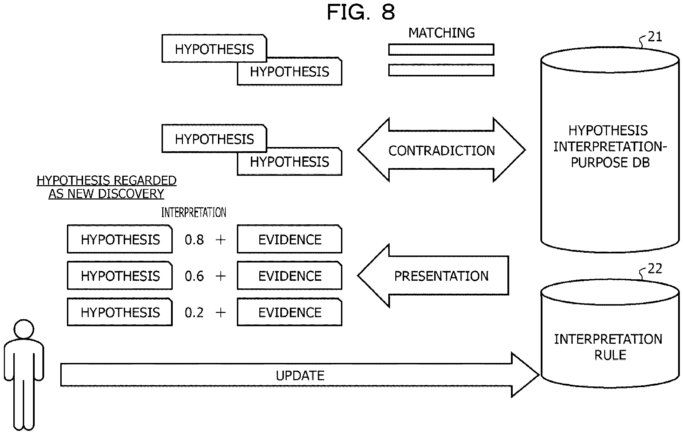

Here, effects according to the first embodiment will be described with reference to FIG. 8. FIG. 8 is a diagram illustrating an effect according to the first embodiment. As illustrated in FIG. 8, the hypothesis interpretation unit 11 interprets a hypothesis to be interpreted, using the hypothesis interpretation-purpose DB 21 and the interpretation rule 22, and presents evidence and a score as an interpretation result. This allows the hypothesis interpretation unit 11 to give interpretation to the hypothesis to be interpreted. As a result, the hypothesis interpretation unit 11 may enable to sort out hypotheses to be interpreted. In addition, the hypothesis interpretation unit 11 may customize and improve the interpretation rule 22 by subjecting the presented evidence and score to confirmation.

As described above, according to the first embodiment, the information processing device 1 receives a hypothesis to be interpreted. The information processing device 1 uses the hypothesis interpretation-purpose DB 21 including, for each piece of knowledge indicating a plurality of resources and a relationship between the resources, basis information serving as a basis of the knowledge and a rule identifier connected with a rule used to interpret a hypothesis, to acquires the basis information and the rule identifier corresponding to the hypothesis to be interpreted. Then, the information processing device 1 uses the interpretation rule 22 including, for each rule identifier, a degree of probability that the rule and the hypothesis coincide with existing knowledge, to acquire the degree of probability of coinciding with the existing knowledge corresponding to the acquired rule identifier. This may allow the information processing device 1 to interpret the hypothesis to be interpreted, using the existing knowledge, in order to sort out the hypothesis.

In addition, according to the first embodiment, the information processing device 1 presents the acquired basis information and degree of probability of coinciding with existing knowledge, for the hypothesis to be interpreted. This may allow the information processing device 1 to subject the hypothesis to be interpreted to sorting. In addition, the hypothesis interpretation unit 11 may customize and improve the interpretation rule 22.

### [Second Embodiment]

Meanwhile, it has been described that the information processing device 1 according to the first embodiment interprets a hypothesis, using the hypothesis interpretation-purpose DB 21 and the interpretation rule 22, and presents evidence and a score as an interpretation result. The hypothesis interpretation-purpose DB 21 used for interpretation has been generated in advance. However, the hypothesis interpretation-purpose DB 21 used for interpretation may be generated using knowledge data between any resources.

Thus, in a second embodiment, a case where the hypothesis interpretation-purpose DB 21 used for interpretation is generated using knowledge data between any resources will be described.

### [Functional Configuration of Information Processing Device]

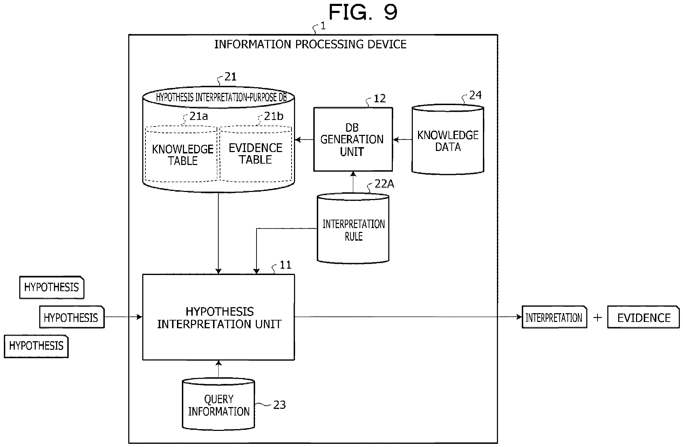

FIG. 9 is a block diagram illustrating an example of a functional configuration of an information processing device according to the second embodiment. Note that, in the functional configuration of the information processing device 1 according to the second embodiment, the same components as those of the information processing device 1 illustrated in FIG. 1 will be indicated by the same reference signs, and the description of these overlapping components and operations will be omitted. The difference between the first embodiment and the second embodiment is that knowledge data 24 and a DB generation unit 12 are added. In addition, the difference between the first embodiment and the second embodiment is that the interpretation rule 22 is changed to an interpretation rule 22A. Note that the knowledge data 24 is an example of a third storage unit.

The knowledge data 24 is a knowledge base indicating knowledge between any resources. The knowledge data 24 stores a plurality of pieces of data in which a subject, an object, and a relationship (predicate) between the subject and the object are treated as one piece of knowledge. In addition, an evidence resource is connected with every one piece of knowledge.

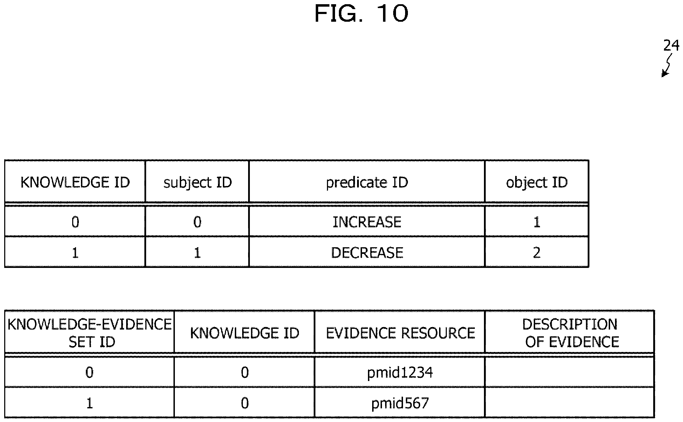

Here, an example of the knowledge data 24 will be described with reference to FIG. 10. FIG. 10 is a diagram illustrating an example of the knowledge data according to the second embodiment. As illustrated in FIG. 10, the knowledge data stores subjectID, predicateID, and objectID in association with the knowledge ID. Furthermore, the knowledge data includes evidence resource data. The evidence resource data stores a knowledge-evidence set ID and the evidence resource in association with the knowledge ID. The subjectID and the objectID include a gene ID as an example. The predicateID includes a relationship between genes. The evidence resource is an identifier for identifying a paper, a document, or a DB serving as a proof of knowledge. Note that the knowledge data 24 is expressed in a table structure, but is not limited thereto and may be expressed in a graph structure by a resource description framework (RDF) or the like, where the evidence resource data may also be stored in association with any partial structure of the graph structure.

As an example, in a case where the knowledge ID is "0", "0" is stored as the subjectID, "increase" is stored as the predicateID, and "1" is stored as the objectID. In addition, in a case where the knowledge ID is "0", two evidence resources are stored. For one evidence resource, "0" is stored as the knowledge-evidence set ID, and "pmid1234" is stored as the evidence resource. For the other evidence resource, "1" is stored as the knowledge-evidence set ID, and "pmid567" is stored as the evidence resource.

Returning to FIG. 9, the interpretation rule 22A includes, for each rule identifier, an interpretation rule for interpreting a hypothesis, a degree of probability of coinciding with existing knowledge, and a query template connected with the knowledge data 24.

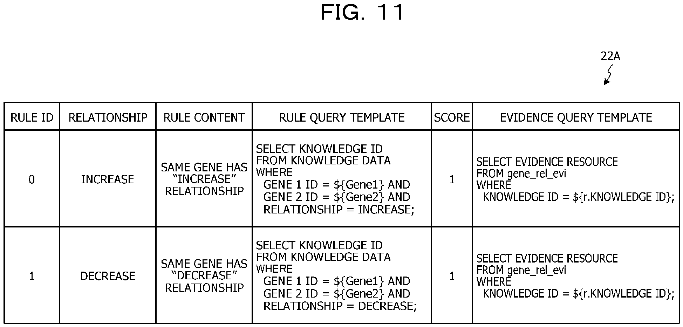

Here, an example of the interpretation rule 22A will be described with reference to FIG. 11. FIG. 11 is a diagram illustrating an example of the interpretation rules according to the second embodiment. Note that, regarding the data configuration of the interpretation rule 22A according to the second embodiment, the description of the same data configuration as that of the interpretation rule 22 illustrated in FIG. 4 will be omitted. The difference between the interpretation rule 22A and the interpretation rule 22 is that a rule query template and an evidence query template are added. The rule query template is a template of a query for connecting a rule with the knowledge ID in the knowledge data 24. The evidence query template is a template of a query for connecting a rule with an evidence resource included in the knowledge data 24. The rule query template and the evidence query template are defined according to the structure of the knowledge data and the type of DB. For example, in a case where the knowledge data is expressed in a graph structure based on RDF, the rule query template and the evidence query template may be written in a format such as SPARQL Protocol and RDF Query Language (SPARQL) that is a query language for RDF.

As an example, in a case where the rule ID is "0", "increase" is stored as the relationship, "same gene has "increase" relationship" is stored as the rule content, and "1" is stored as the score. Additionally, the rule query template and the evidence query template are stored.

Returning to FIG. 9, the DB generation unit 12 generates a hypothesis interpretation-purpose DB 21 from the knowledge data 24 and the interpretation rule 22A. For example, the DB generation unit 12 acquires a resource set that is a target of a hypothesis. The DB generation unit 12 performs the following process on each rule in the interpretation rule 22A with respect to the acquired resource set. The DB generation unit 12 generates a query from the resource set and the rule query template corresponding to one rule ID in the interpretation rule 22A. Then, the DB generation unit 12 inquires of the knowledge data 24 about the generated query and acquires an inquiry result as to whether or not there is a knowledge ID that matches the rule. In a case where the inquiry result is a result that there is a knowledge ID matching the rule, the DB generation unit 12 generates a query from the knowledge ID and the evidence query template corresponding to the same rule ID. Then, the DB generation unit 12 inquires of the knowledge data 24 about the generated query and acquires an evidence resource corresponding to the knowledge indicated by the knowledge ID. The DB generation unit 12 then connects the relationship included in the rule with the resource set and updates a knowledge table 21a of the hypothesis interpretation-purpose DB 21. At the time of updating, a knowledge ID is allocated. Additionally, the DB generation unit 12 updates an evidence table 21b of the hypothesis interpretation-purpose DB 21 by connecting the evidence resource with the rule ID and the allocated knowledge ID. In this manner, the DB generation unit 12 performs the DB generation process for each resource set in the list of resource sets as targets of hypotheses.

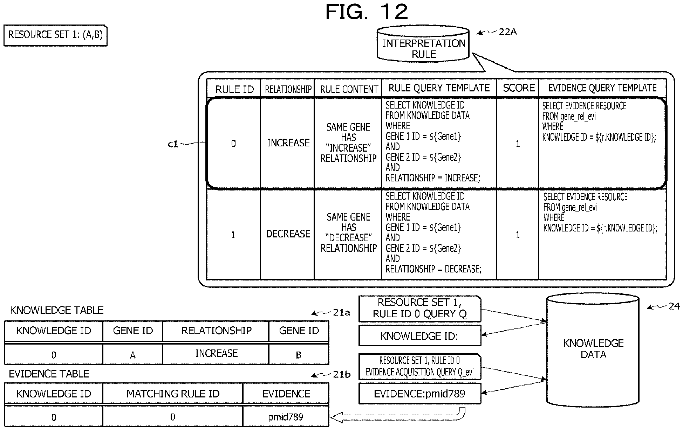

FIG. 12 is a diagram illustrating an example of DB generation according to the second embodiment. As illustrated in FIG. 12, it is assumed that the DB generation unit 12 acquires "A, B" as one resource set.

The DB generation unit 12 selects one rule from the interpretation rule 22A. Here, the rule of which the rule ID indicates "0" is selected (reference sign c1). Then, the DB generation unit 12 determines whether or not the resource set matches the rule. That is, the DB generation unit 12 generates a query from the rule query template for the rule and the resource set. Here, the query is generated by substituting "A" and "B" into the "$(Gene1)" and "$(Gene2)" portions of the rule query template.

Then, the DB generation unit 12 inquires of the knowledge data 24 about the generated query and acquires an inquiry result as to whether or not there is a knowledge ID that matches the rule. Here, an inquiry result in which the knowledge ID indicates "1234" is acquired. In other words, the resource set matches the rule, and there is a knowledge ID that matches the rule.

Then, in a case where the inquiry result is a result that there is the knowledge ID matching the rule, the DB generation unit 12 generates an evidence acquisition query from the knowledge ID and the evidence query template corresponding to the same rule ID. Here, the evidence acquisition query is generated by substituting "1234" into the "knowledge ID" portion of the evidence query template.

Then, the DB generation unit 12 inquires of the knowledge data 24 about the generated evidence acquisition query and acquires an evidence resource corresponding to the knowledge indicated by the knowledge ID. Here, "pmid789" is acquired as the evidence resource.

Then, the DB generation unit 12 connects the relationship included in the rule and the newly allocated knowledge ID with the resource set and updates the knowledge table 21a of the hypothesis interpretation-purpose DB 21. Here, in the knowledge table 21a, in a case where the knowledge ID is "0", "A" is updated as the gene ID, "increase" is updated as the relationship, and "B" is updated as the gene ID.

Additionally, the DB generation unit 12 updates the evidence table 21b of the hypothesis interpretation-purpose DB 21 by connecting the evidence resource with the rule ID and the allocated knowledge ID. Here, in the evidence table 21b, "pmid789" is updated as the evidence in a case where the knowledge ID is "0" and the matching rule ID is "0".

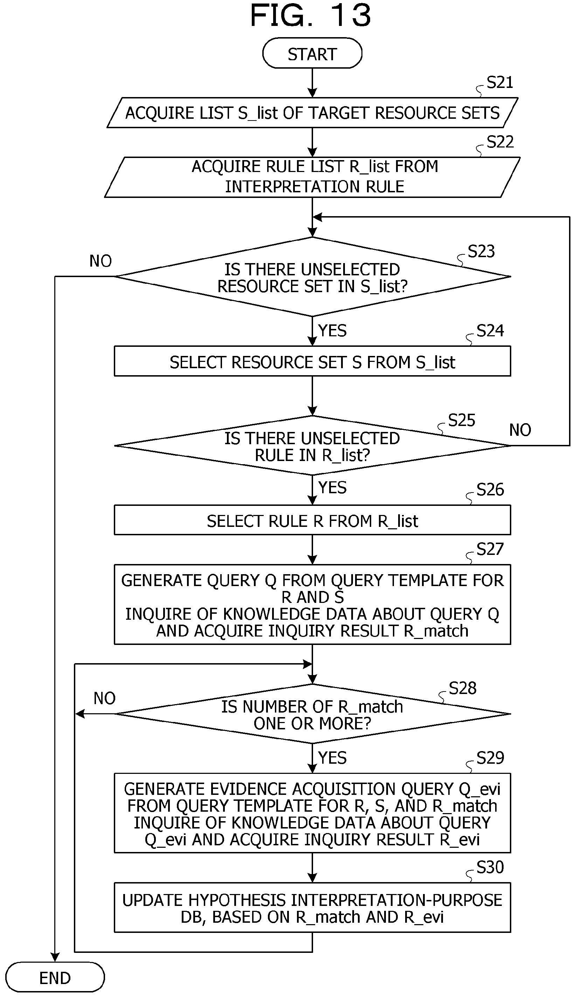

FIG. 13 is a diagram illustrating an example of a flowchart of the DB generation process according to the second embodiment.

As illustrated in FIG. 13, the DB generation unit 12 acquires a list S_list of target resource sets (step S21). Note that the list of the target resource sets may be, for example, a list generated in advance or a list generated from a hypothesis extracted in a certain hypothesis extraction phase.

Then, the DB generation unit 12 acquires a rule list R_list from the interpretation rule 22A (step S22). The DB generation unit 12 determines whether or not there is an unselected resource set in the list S_list of resource sets (step S23). In a case where it is determined that there is an unselected resource set in the list S_list of resource sets (step S23; Yes), the DB generation unit 12 selects a resource set S from the list S_list of resource sets (step S24).

Then, the DB generation unit 12 determines whether or not there is an unselected rule in the rule list R_list (step S25). In a case where it is determined that there is no unselected rule in the rule list R_list (step S25; No), the DB generation unit 12 proceeds to step S23 to select a next resource set.

On the other hand, in a case where it is determined that there is an unselected rule in the rule list R_list (step S25; Yes), the DB generation unit 12 selects a rule R from the rule list R_list (step S26). The DB generation unit 12 generates a query Q from the query template for the rule R and the resource set S, inquires of the knowledge data 24 about the query Q, and acquires an inquiry result Rmatch (step S27). Then, the DB generation unit 12 determines whether or not the number of inquiry results R match is one or more (step S28).

In a case where it is determined that the number of inquiry results R match is not one or more (step S28; No), the DB generation unit 12 proceeds to step S25 to select a next rule.

On the other hand, in a case where it is determined that the number of inquiry results R match is one or more (step S28; Yes), the DB generation unit 12 generates an evidence acquisition query Q_evi from the query template for the rule R, the resource set S, and the inquiry result R match, inquires of the knowledge data 24 about the evidence acquisition query Q_evi, and acquires an inquiry result R_evi (step S29).

Then, the DB generation unit 12 updates the hypothesis interpretation-purpose DB 21, based on the inquiry results R_match and R_evi (step S30). That is, the DB generation unit 12 updates the knowledge table 21a and the evidence table 21b of the hypothesis interpretation-purpose DB 21. Then, the DB generation unit 12 proceeds to step S25 to select a next rule.

In step S23, in a case where the DB generation unit 12 determines that there is no unselected resource set in the list S_list of resource sets (step S23; No), the DB generation unit 12 ends the DB generation process.

### [Effects of Second Embodiment]

As described above, according to the second embodiment, the information processing device 1 further receives a set of resources and acquires the basis information for the set of resources from the knowledge data 24 in which the basis information serving as a basis of the knowledge is connected with each piece of knowledge including the subject, the object, and the predicate indicating the relationship between the subject and the object, based on the rule included in the interpretation rule 22A. Then, for the set of resources, the information processing device 1 adds, to the hypothesis interpretation-purpose DB 21, information including the relationship between the resources obtained from the rule in the interpretation rule 22A, the basis information, and the rule identifier connected with the rule. This may allow the information processing device 1 to generate the hypothesis interpretation-purpose DB 21 used when interpreting a hypothesis by using the knowledge data 24 and the interpretation rule 22A.

### [Third Embodiment]

Meanwhile, it has been described that the information processing device 1 according to the first embodiment interprets a hypothesis, using the hypothesis interpretation-purpose DB 21 and the interpretation rule 22, and presents evidence and a score as an interpretation result. In addition, it has been described that the information processing device 1 according to the second embodiment generates the hypothesis interpretation-purpose DB 21 from the knowledge data 24 and the interpretation rule 22A. However, the information processing device 1 is not limited thereto and may interpret a hypothesis, using the interpretation rule 22A and the knowledge data 24 instead of the hypothesis interpretation-purpose DB 21, and present evidence and a score as an interpretation result.

Thus, in a third embodiment, an information processing device 1 that interprets a hypothesis, using knowledge data 24 and an interpretation rule 22A, and presents evidence and a score as an interpretation result will be described.

### [Functional Configuration of Information Processing Device]

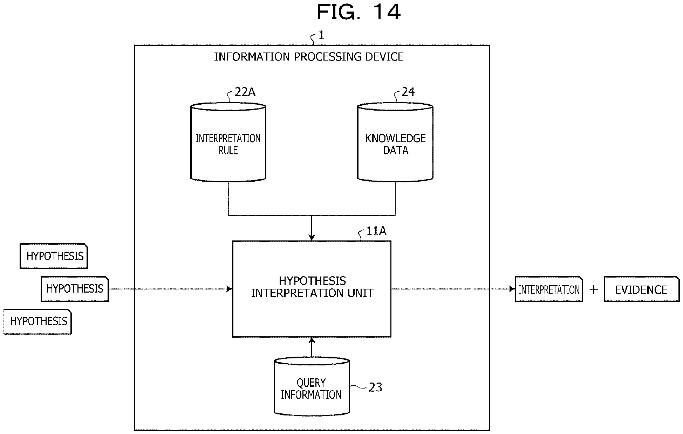

FIG. 14 is a block diagram illustrating an example of a functional configuration of the information processing device according to the third embodiment. Note that, in the functional configuration of the information processing device 1 according to the third embodiment, the same components as those of the information processing device 1 illustrated in FIG. 9 will be indicated by the same reference signs, and the description of these overlapping components and operations will be omitted. The difference between the second embodiment and the third embodiment is that the hypothesis interpretation-purpose DB 21 and the DB generation unit 12 are deleted. In addition, the difference between the second embodiment and the third embodiment is that the hypothesis interpretation unit 11 is changed to a hypothesis interpretation unit 11A.

The hypothesis interpretation unit 11A interprets a hypothesis, using knowledge data 24 and an interpretation rule 22A. For example, after acquiring a hypothesis to be interpreted, the hypothesis interpretation unit 11A performs the following process on each rule in the interpretation rule 22A with respect to the acquired hypothesis to be interpreted. The hypothesis interpretation unit 11A generates a query from the hypothesis and the rule query template corresponding to one rule ID in the interpretation rule 22A. Then, the hypothesis interpretation unit 11A inquires of the knowledge data 24 about the generated query and acquires an inquiry result as to whether or not there is a knowledge ID that matches the rule. In a case where the inquiry result is a result that there is a knowledge ID matching the rule, the hypothesis interpretation unit 11A generates a query from the knowledge ID and the evidence query template corresponding to the same rule ID. Then, the hypothesis interpretation unit 11A inquires of the knowledge data 24 about the generated query and acquires an evidence resource corresponding to the knowledge indicated by the knowledge ID. The hypothesis interpretation unit 11A then holds information in which the knowledge ID, the evidence, the matching rule ID, and the score are associated, as a matching result list.

Then, the hypothesis interpretation unit 11A aggregates the held matching result list. Note that, as an example, the hypothesis interpretation unit 11A can simply prioritize information having a higher score in information included in the matching result list at the time of aggregation, but as another example, all the evidence may be written together with the scores as an average value, or the aggregation is not limited thereto.

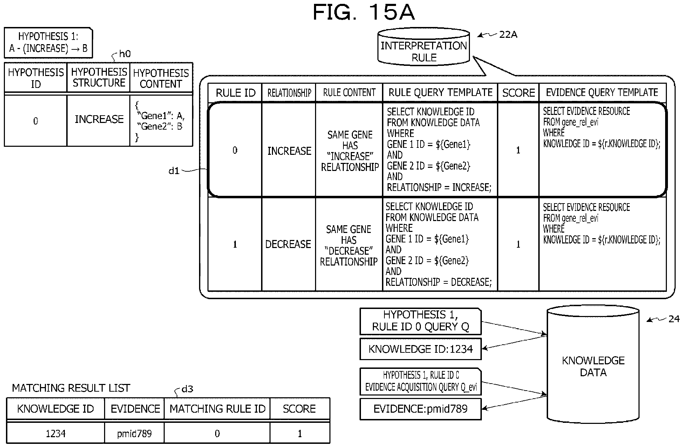

FIGs. 15A to 15C are diagrams illustrating an example of hypothesis interpretation according to the third embodiment. As illustrated in FIG. 15A, it is assumed that hypothesis interpretation unit 11A acquires "A - (increase) → B" (a relationship that B increases as A increases) as a hypothesis. As an example, the hypothesis has the content indicated by the reference sign h0, which is a case where the hypothesis structure is "increase".

The hypothesis interpretation unit 11A selects one rule from the interpretation rule 22A. Here, the rule of which the rule ID indicates "0" is selected (reference sign d1). Then, the hypothesis interpretation unit 11A determines whether or not the hypothesis matches the rule. That is, the hypothesis interpretation unit 11A generates a query from the rule query template for the rule and the hypothesis. Here, the query is generated by substituting "A" and "B" into the "$(Gene1)" and "$(Gene2)" portions of the rule query template.

Then, the hypothesis interpretation unit 11A inquires of the knowledge data 24 about the generated query and acquires an inquiry result as to whether or not there is a knowledge ID that matches the rule. Here, an inquiry result in which the knowledge ID indicates "1234" is acquired. In other words, the hypothesis matches the rule, and there is a knowledge ID that matches the rule.

Then, in a case where the inquiry result is a result that there is the knowledge ID matching the rule, the hypothesis interpretation unit 11A generates an evidence acquisition query from the knowledge ID and the evidence query template corresponding to the same rule ID. Here, the evidence acquisition query is generated by substituting "1234" into the "knowledge ID" portion of the evidence query template.

Then, the hypothesis interpretation unit 11A inquires of the knowledge data 24 about the generated evidence acquisition query and acquires an evidence resource corresponding to the knowledge indicated by the knowledge ID. Here, "pmid789" is acquired as the evidence resource.

The hypothesis interpretation unit 11A then holds information in which the knowledge ID, the evidence, the matching rule ID, and the score are associated, as a matching result list. Here, the matching result list is information indicated by the reference sign d3.

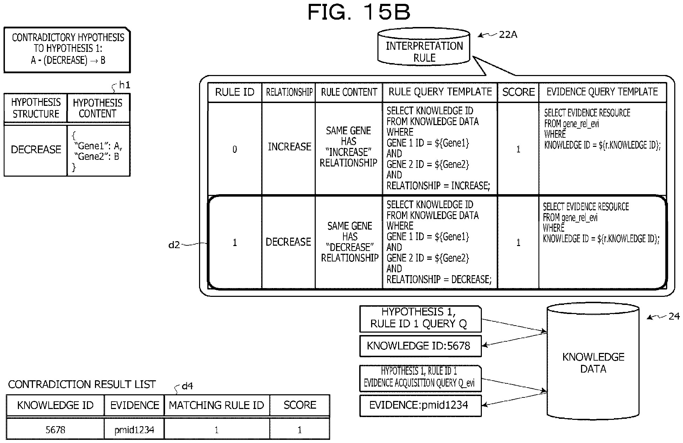

Next, as illustrated in FIG. 15B, it is assumed that the hypothesis interpretation unit 11A accepts an input of a contradictory hypothesis "A - (decrease) → B" (a relationship that B decreases as A decreases) to "A - (increase) → B" acquired as a hypothesis. As an example, the contradictory hypothesis has the content indicated by the reference sign h1, which is a case where the hypothesis structure is "decrease" contradicting the hypothesis.

The hypothesis interpretation unit 11A selects one rule from the interpretation rule 22A. Here, the rule of which the rule ID indicates "1" is selected (reference sign d2). Then, the hypothesis interpretation unit 11A determines whether or not the hypothesis matches the rule. That is, the hypothesis interpretation unit 11A generates a query from the rule query template for the rule and the hypothesis. Here, the query is generated by substituting "A" and "B" into the "$(Gene1)" and "$(Gene2)" portions of the rule query template.

Then, the hypothesis interpretation unit 11A inquires of the knowledge data 24 about the generated query and acquires an inquiry result as to whether or not there is a knowledge ID that matches the rule. Here, an inquiry result in which the knowledge ID indicates "5678" is acquired. In other words, the contradictory hypothesis matches the rule, and there is a knowledge ID that matches the rule.

Then, in a case where the inquiry result is a result that there is the knowledge ID matching the rule, the hypothesis interpretation unit 11A generates an evidence acquisition query from the knowledge ID and the evidence query template corresponding to the same rule ID. Here, the evidence acquisition query is generated by substituting "5678" into the "knowledge ID" portion of the evidence query template.

Then, the hypothesis interpretation unit 11A inquires of the knowledge data 24 about the generated evidence acquisition query and acquires an evidence resource corresponding to the knowledge indicated by the knowledge ID. Here, "pmid1234" is acquired as the evidence resource.

Then, the hypothesis interpretation unit 11A holds information in which the knowledge ID, the evidence, the matching rule ID, and the score are associated, as a contradiction result list. Here, the contradiction result list is information indicated by the reference sign d4.

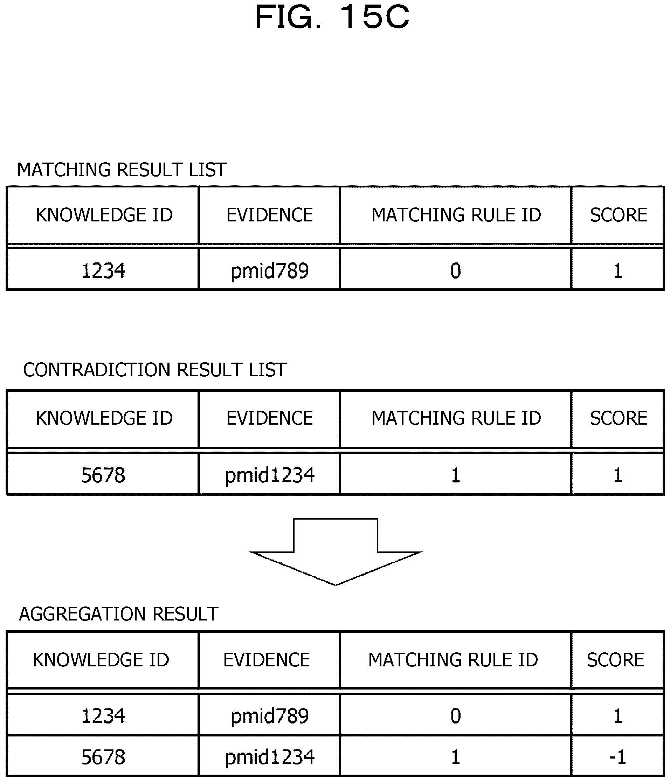

Then, as illustrated in FIG. 15C, the hypothesis interpretation unit 11A aggregates the held matching result list and contradiction result list and holds the aggregated matching result list and contradiction result list in an aggregation result. Here, the aggregation result is held in the order of the matching result list and the contradiction result list in the descending order of scores. Here, the aggregation result of the knowledge ID, the evidence, and the matching rule ID is held in the order of scores of "1" and "-1". The last score of the aggregation result is negative because it is a score for contradictory hypothesis.

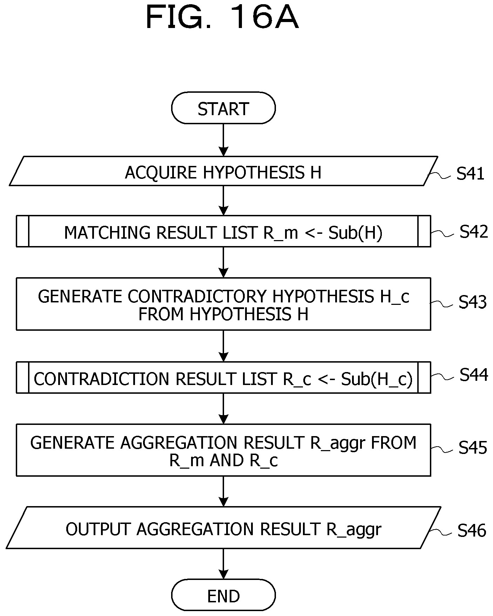

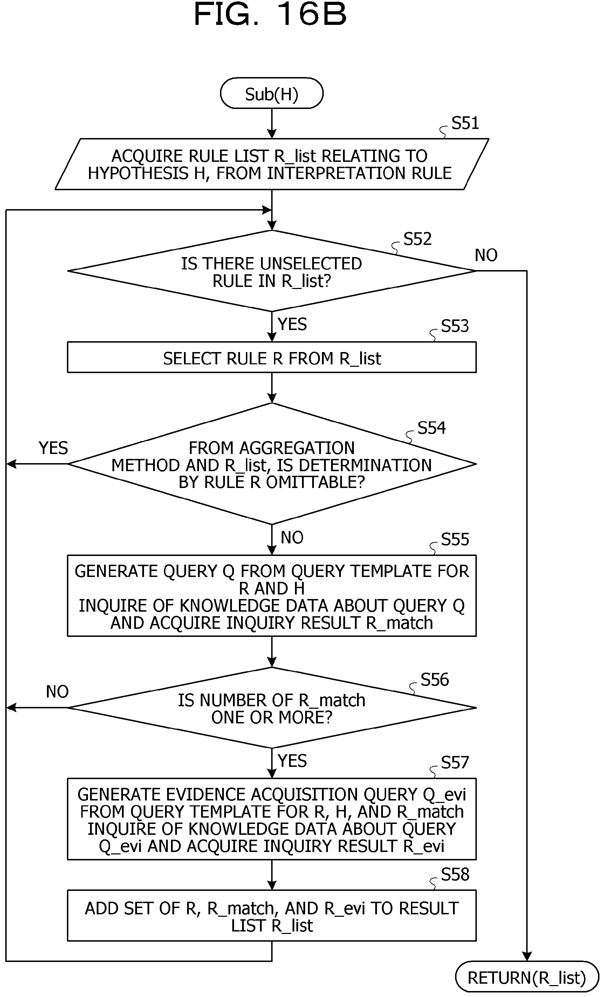

FIGs. 16A and 16B are diagrams illustrating an example of a flowchart of a hypothesis interpretation process according to the third embodiment.

As illustrated in FIG. 16A, the hypothesis interpretation unit 11A acquires a hypothesis H (step S41). Then, the hypothesis interpretation unit 11A calls a process of inputting the hypothesis H and generating a matching result list R_m (step S42). Note that the process of generating the result list from the hypothesis will be described later.

Subsequently, the hypothesis interpretation unit 11A generates a contradicting hypothesis H_c from the hypothesis H (step S43). Then, the hypothesis interpretation unit 11A calls a process of inputting the contradicting hypothesis H c and generating a contradiction result list R c (step S44). Note that the process of generating the result list from the hypothesis will be described later.

Then, the hypothesis interpretation unit 11A generates an aggregation result R_aggr from the matching result list R_m and the contradiction result list R c (step S45).

The hypothesis interpretation unit 11A then outputs the aggregation result R_aggr (step S46). Then, the hypothesis interpretation unit 11A ends.

In FIG. 16B, the hypothesis interpretation unit 11A executes a process of generating a matching or contradiction result list as follows. When the hypothesis H is input, the hypothesis interpretation unit 11A acquires a rule list R_list relating to the hypothesis H, from the interpretation rule 22A (step S51). For example, in a case where the hypothesis is "A - (increase) → B", the hypothesis interpretation unit 11A acquires the rule list in which the relationship indicates the increase, from the interpretation rule 22A.

The hypothesis interpretation unit 11A determines whether or not there is an unselected rule in the rule list R_list (step S52). In a case where it is determined that there is an unselected rule in the rule list R_list (step S52; Yes), the hypothesis interpretation unit 11A selects a rule R from the rule list R_list (step S53).

Then, the hypothesis interpretation unit 11A determines whether or not the determination by the rule R is omittable, from the aggregation method and the rule list R_list (step S54). In a case where it is determined that the determination by the rule R is omittable (step S54; Yes), the hypothesis interpretation unit 11A proceeds to step S52 to select a next rule.

On the other hand, in a case where it is determined that the determination by the rule R is not omittable (step S54; No), the hypothesis interpretation unit 11A generates a query Q from the rule query template for the rule R and the input hypothesis H. Then, the hypothesis interpretation unit 11A inquires of the knowledge data 24 about the generated query Q and acquires an inquiry result R match (step S55).

Then, the hypothesis interpretation unit 11A determines whether or not the number of inquiry results R match is one or more (step S56) . In a case where it is determined that the number of inquiry results R match is not one or more (step S56; No), the hypothesis interpretation unit 11A proceeds to step S52 to select a next rule.

On the other hand, in a case where it is determined that the number of inquiry results R match is one or more (step S56; Yes), the hypothesis interpretation unit 11A generates an evidence acquisition query Q_evi from the evidence query template for the rule R, the input hypothesis H, and the inquiry result R_match. Then, the hypothesis interpretation unit 11A inquires of the knowledge data 24 about the evidence acquisition query Q_evi and acquires an inquiry result R_evi (step S57).

Then, the hypothesis interpretation unit 11A adds a set of the rule R, the inquiry result R_match, and the inquiry result R_evi to the result list R_list (step S58). The hypothesis interpretation unit 11A then proceeds to step S52 to select a next rule.

In step S52, in a case where the hypothesis interpretation unit 11A determines that there is no unselected rule in the rule list R_list (step S52; No), the hypothesis interpretation unit 11A returns the result list R_list to the caller.

### [Effects of Third Embodiment]

As described above, according to the third embodiment, the information processing device 1 receives a hypothesis to be interpreted and determines whether or not knowledge corresponding to the hypothesis to be interpreted exists in the knowledge data 24 in which the basis information serving as a basis of the knowledge is connected with each piece of the knowledge that includes a subject, an object, and a predicate indicating a relationship between the subject and the object, using a rule that relates to the hypothesis to be interpreted and is included in the interpretation rule 22A including, for each rule identifier, a rule used to interpret the hypothesis and a probability that the hypothesis coincides with existing knowledge. Then, in a case where it is determined that knowledge corresponding to the hypothesis to be interpreted exists in the knowledge data 24, the information processing device 1 acquires the basis information corresponding to the knowledge and the probability of coinciding with the existing knowledge corresponding to the rule relating to the hypothesis to be interpreted. This may allow the information processing device 1 to interpret a hypothesis to be interpreted, using the knowledge data 24 and the interpretation rule 22A, and present evidence and a score as an interpretation result without generating the hypothesis interpretation-purpose DB 21.

### [Fourth Embodiment]

Meanwhile, it has been described that the information processing device 1 according to the first embodiment interprets a hypothesis, using the hypothesis interpretation-purpose DB 21 and the interpretation rule 22, and presents evidence and a score as an interpretation result. It has been described that the information processing device 1 according to the second embodiment generates the hypothesis interpretation-purpose DB 21 used for interpretation, using the knowledge data 24 and the interpretation rule 22A. Here, the knowledge data 24 is a knowledge base indicating existing knowledge between any resources. Such existing knowledge exists in disjointed formats such as a paper, a document, and a DB and also includes non-structural data and non-machine-readable data. Therefore, the information processing device 1 may generate the knowledge data 24 by utilizing a relationship extraction process from a natural language with regard to papers, documents, and the like, and utilizing a process for DB integration with regard to DBs and the like.

Thus, in a fourth embodiment, an information processing device 1 that generates knowledge data 24 by utilizing a relationship extraction process from a natural language and a process for DB integration will be described.

### [Functional Configuration of Information Processing Device]

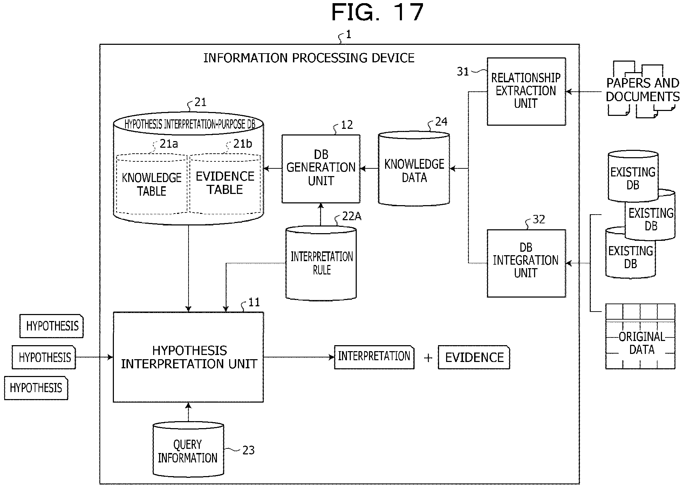

FIG. 17 is a block diagram illustrating an example of a functional configuration of the information processing device according to the fourth embodiment. Note that, in the functional configuration of the information processing device 1 according to the fourth embodiment, the same components as those of the information processing device 1 illustrated in FIG. 9 will be indicated by the same reference signs, and the description of these overlapping components and operations will be omitted. The difference between the second embodiment and the fourth embodiment is that a relationship extraction unit 31 and a DB integration unit 32 are added.

The relationship extraction unit 31 extracts a relationship from a natural language. For example, a relationship extraction rule is predefined. The relationship extraction unit 31 determines whether or not the target paper and document match a predefined relationship extraction rule. Then, in a case where the target paper and document match the relationship extraction rule, the relationship extraction unit 31 generates relationship data with the paper and document matching the rule as evidence resources. The relationship data is one piece of knowledge. Then, the relationship extraction unit 31 saves the evidence resources in the knowledge data 24 in connection with one piece of knowledge. Note that a method of implementing the relationship extraction is not limited, and machine learning, a language model, or the like may be used, or any existing technique may be used.

The DB integration unit 32 integrates a plurality of existing DBs. For example, a DB integration rule is predefined. As an example, the DB integration rule is a rule for integrating entities or a rule for unifying items. The DB integration unit 32 integrates a plurality of target existing DBs in accordance with a predefined DB integration rule. Then, the DB integration unit 32 generates knowledge between resources with the integrated DB as evidence resources. The DB integration unit 32 then saves the evidence resource in the knowledge data 24 in connection with one piece of knowledge.

### [Effects of Fourth Embodiment]

As described above, according to the fourth embodiment, the information processing device 1 generates the knowledge data 24 that treats the target document as the basis information, using a predetermined relationship extraction process from a natural language. Then, the information processing device 1 integrates a plurality of target DBs, using a predetermined integration process for integrating a plurality of existing DBs, and generates the knowledge data 24 that treats the integrated DB as the basis information. This may allow the information processing device 1 to generate the knowledge data 24 from documents or DBs existing in disjointed formats.

### [Fifth Embodiment]

Meanwhile, it has been described that the information processing device 1 according to the first embodiment interprets a hypothesis, using the hypothesis interpretation-purpose DB 21 and the interpretation rule 22, and presents evidence and a score as an interpretation result. It has been described that the information processing device 1 according to the second embodiment generates the hypothesis interpretation-purpose DB 21 used for interpretation, using the knowledge data 24 and the interpretation rule 22A. It has been described that the information processing device 1 according to the fourth embodiment generates the knowledge data 24 by utilizing a relationship extraction process from a natural language and a process for DB integration. Here, humans often create rules regarding the interpretation rules 22 and 22A used for interpretation. When a human creates a rule, for example, there is a disadvantage that it takes time.

Thus, in a fifth embodiment, an information processing device 1 that generates an interpretation rule 22A will be described.

### [Functional Configuration of Information Processing Device]

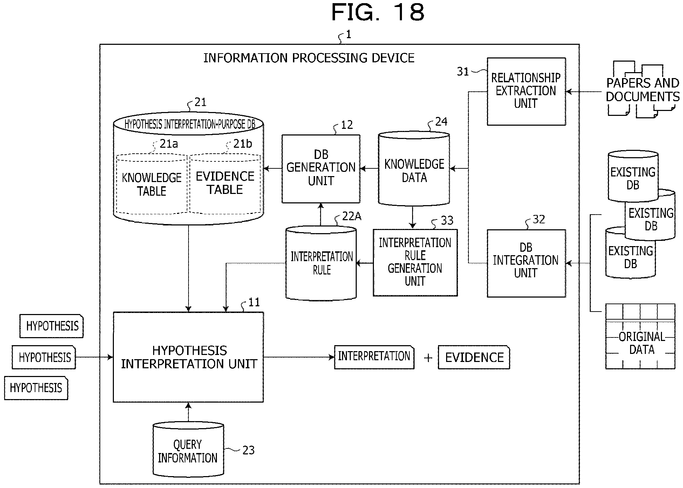

FIG. 18 is a block diagram illustrating an example of a functional configuration of the information processing device according to the fifth embodiment. Note that, in the functional configuration of the information processing device 1 according to the fifth embodiment, the same components as those of the information processing device 1 illustrated in FIG. 17 will be indicated by the same reference signs, and the description of these overlapping components and operations will be omitted. The difference between the fourth embodiment and the fifth embodiment is that an interpretation rule generation unit 33 is added.

The interpretation rule generation unit 33 generates the interpretation rule 22A. For example, the interpretation rule generation unit 33 acquires a list of resource sets and also designates a relationship between resources to be extracted. The interpretation rule generation unit 33 extracts a feature table corresponding to the resource sets included in the list and the designated relationship from the knowledge data 24, for example. As an example, in a case where the knowledge data 24 is a graph DB, the interpretation rule generation unit 33 can extract a feature from a graph pattern of graph structure between entities.

Then, with the feature table as an input, the interpretation rule generation unit 33 calculates a list of scores of respective features included in the feature table, by applying explainable artificial intelligence (AI) that treats a variable representing the presence or absence of the designated relationship as an objective variable. The explainable artificial intelligence (AI) mentioned here refers to AI configured to calculate a score (confidence) of each feature (or a product of features) with respect to the objective variable. Examples of the explainable AI include frequent pattern mining, logistic regression, and the like.

Then, the interpretation rule generation unit 33 generates the interpretation rule 22A, based on the calculated list of the scores of respective features. As an example, the interpretation rule generation unit 33 adds the designated relationship, the feature, and the score to the interpretation rule 22A in association with each other.

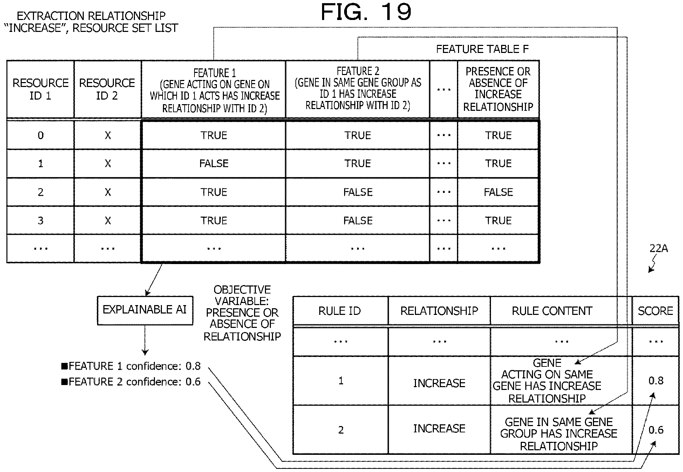

FIG. 19 is a diagram illustrating an example of interpretation rule generation according to the fifth embodiment. As illustrated in FIG. 19, it is assumed that the interpretation rule generation unit 33 acquires a resource set list and "increase" as the relationship to be extracted (extraction relationship).

The interpretation rule generation unit 33 extracts, from the knowledge data 24, a feature table F corresponding to the resource sets included in the resource set list and the extraction relationship "increase".

Then, with the extracted feature table F as an input, the interpretation rule generation unit 33 calculates a list of scores of respective features included in the feature table F by applying the explainable AI that treats a variable representing the presence or absence of the extraction relationship "increase" as an objective variable. Here, the score (confidence) is calculated as "0.8" for a feature 1, and the score (confidence) is calculated as "0.6" for a feature 2.

Then, the interpretation rule generation unit 33 generates the interpretation rule 22A, based on the calculated list of the scores of respective features. Here, regarding the interpretation rule 22A, the interpretation rule generation unit 33 adds, to the interpretation rule 22A, the extraction relationship "increase" as a relationship item, the features 1 and 2 as rule content items, and scores (confidence) corresponding to the features 1 and 2 as score items.

Then, the interpretation rule generation unit 33 similarly handles other extraction relationships as well to generate the interpretation rule 22A.

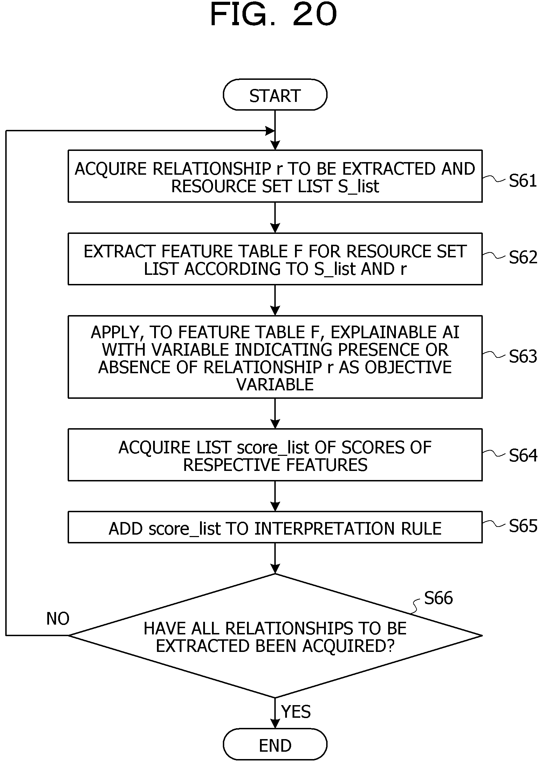

FIG. 20 is a diagram illustrating an example of a flowchart of an interpretation rule generation process according to the fifth embodiment. As illustrated in FIG. 20, the interpretation rule generation unit 33 acquires a relationship r to be extracted and a resource set list S_list (step S61). Then, the interpretation rule generation unit 33 extracts the feature table F for the resource set list, using, for example, the knowledge data 24, according to the resource set list S_list and the relationship r to be extracted (step S62) .

Then, the interpretation rule generation unit 33 applies, to the feature table F, the explainable AI with a variable indicating the presence or absence of the relationship r as an objective variable (step S63). The interpretation rule generation unit 33 then acquires a list score list of scores of respective features (step S64). Then, the interpretation rule generation unit 33 adds the list score_list of scores of respective features to the interpretation rule 22A (step S65) .

The interpretation rule generation unit 33 then determines whether or not all the relationships to be extracted have been acquired (step S66). In a case where it is determined that all the relationships to be extracted have not been acquired (step S66; No), the interpretation rule generation unit 33 proceeds to step S61 to acquire a next relationship to be extracted.

On the other hand, in a case where it is determined that all the relationships to be extracted have been acquired (step S66; Yes), the interpretation rule generation unit 33 ends the interpretation rule generation process.

### [Effects of Fifth Embodiment]

As described above, according to the fifth embodiment, the information processing device 1 further receives the list of the sets of resources and the relationship to be extracted. The information processing device 1 extracts a feature corresponding to the set of resources included in the list and the relationship between to be extracted, from the knowledge data 24. The information processing device 1 accepts an input of the extracted features and outputs the confidence of the features by applying the explainable AI that treats a variable indicating the presence or absence of the relationship to be extracted, as an objective variable. Then, the information processing device 1 adds, to the interpretation rule 22A, information in which the extracted features is treated as a rule, which is information in which the output confidence of the feature is treated as the probability of coinciding with existing knowledge, and which is information with which the relationship to be extracted, is associated. This may allow the information processing device 1 to generate the interpretation rule 22A by using the knowledge data 24.

### [Sixth Embodiment]

Meanwhile, it has been described that the information processing device 1 according to the first embodiment interprets a hypothesis, using the hypothesis interpretation-purpose DB 21 and the interpretation rule 22, and presents evidence and a score as an interpretation result. Thus, in a sixth embodiment, an information processing device 1 that presents an interpretation result will be described.

### [Functional Configuration of Information Processing Device]

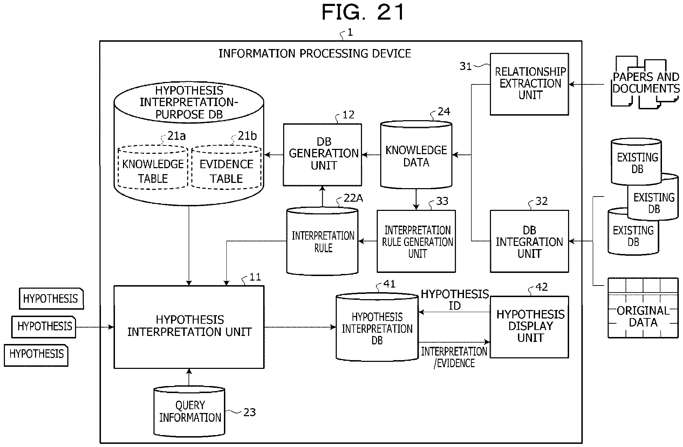

FIG. 21 is a block diagram illustrating an example of a functional configuration of the information processing device according to the sixth embodiment. Note that, in the functional configuration of the information processing device 1 according to the sixth embodiment, the same components as those of the information processing device 1 illustrated in FIG. 18 will be indicated by the same reference signs, and the description of these overlapping components and operations will be omitted. The difference between the fifth embodiment and the sixth embodiment is that a hypothesis interpretation DB 41 and a hypothesis display unit 42 are added. Note that the hypothesis interpretation DB 41 is an example of a fourth storage unit.

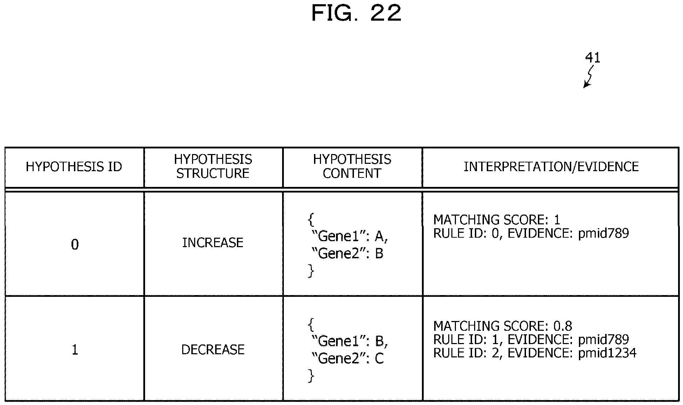

The hypothesis interpretation DB 41 stores an interpretation result for a hypothesis interpreted by a hypothesis interpretation unit 11. Here, an example of the hypothesis interpretation DB 41 will be described with reference to FIG. 22. FIG. 22 is a diagram illustrating an example of the hypothesis interpretation DB according to the sixth embodiment.

As illustrated in FIG. 22, the hypothesis interpretation DB 41 stores a hypothesis ID, a hypothesis structure, hypothesis content, and interpretation/evidence in association with each other. The hypothesis ID is an ID for uniquely identifying a hypothesis to be interpreted. The hypothesis structure corresponds to the relationship between resources indicated by the hypothesis. The hypothesis content corresponds to a plurality of resources connected with the hypothesis structure. The interpretation/evidence is set with a rule ID and a score of a rule in an interpretation rule 22A used to interpret the hypothesis, and evidence connected with the rule.

As an example, in a case where the hypothesis ID is "0", "increase" is stored as the hypothesis structure, "{"Gene1": A, "Gene2": B}" is stored as the hypothesis content, and "matching score: 1, rule ID: 0, evidence: pmid789" is stored as the interpretation/evidence.

The hypothesis display unit 42 displays an interpretation result for a hypothesis. For example, when receiving a hypothesis ID instructed by a user interface (UI), the hypothesis display unit 42 extracts the interpretation/evidence corresponding to the hypothesis ID from the hypothesis interpretation DB 41. Then, the hypothesis display unit 42 displays the extracted interpretation/evidence on a screen. Note that the hypothesis display unit 42 can perform, for example, filtering and sorting according to interpretation results for hypotheses by UIs.

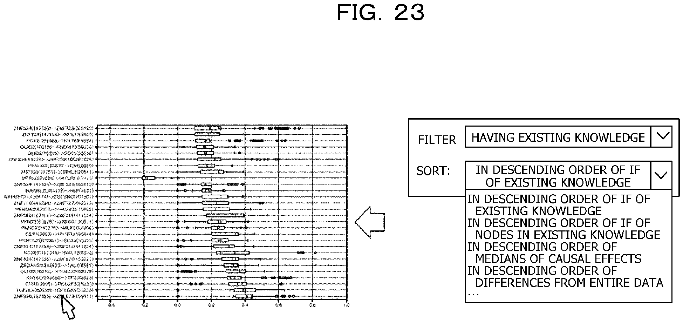

FIG. 23 is a diagram illustrating an example of a screen image. The right diagram of FIG. 23 displays a menu indicating one UI. In the menu, a list box for filtering and a list box for sorting are represented. Here, "having existing knowledge" is selected as a filter. "In descending order of IF of existing knowledge" is selected as sorting. The hypothesis display unit 42 filters and extracts the interpretation results for hypotheses from the hypothesis interpretation DB 41 according to the selected filter "having existing knowledge". The hypothesis display unit 42 sorts the interpretation results extracted by filtering "in descending order of IF of existing knowledge" as the selected sorting. Then, the hypothesis display unit 42 displays the filtered and sorted interpretation results on the screen.

The left diagram of FIG. 23 represents an interpretation result displayed by the hypothesis display unit 42. Here, what score the hypothesis has is represented.

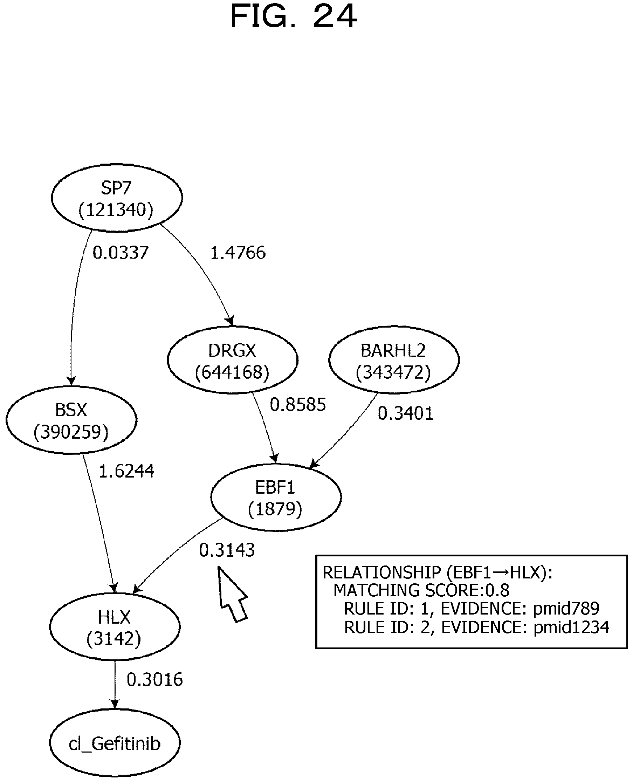

FIG. 24 is a diagram illustrating another example of a screen image. The left diagram of FIG. 24 represents a graph in which hypotheses are combined. For example, the node such as "EBF1" or "HLX" indicates a gene ID. The arrow and numerical value between "EBF1" and "HLX" indicate the relationship between the genes. "EBF1 → HLX" denotes one hypothesis and has a unique hypothesis ID.

When, for example, the arrow portion of such a graph is clicked, the hypothesis display unit 42 receives the hypothesis ID corresponding to the clicked arrow portion. The hypothesis display unit 42 extracts the interpretation/evidence corresponding to the hypothesis ID, which has been received from the hypothesis interpretation DB 41. Then, the hypothesis display unit 42 displays the extracted interpretation/evidence on the screen. Here, the interpretation/evidence "matching score: 0.8; rule ID: 1, evidence: pmid789; rule ID: 2, evidence: pmid1234" is displayed for the hypothesis "EBF1 → HLX".

### [Effects of Sixth Embodiment]

As described above, according to the sixth embodiment, the information processing device 1 extracts and presents the basis information and the probability of coinciding with existing knowledge for the target hypothesis, from the hypothesis interpretation DB 41, based on the user interface. This may allow the information processing device 1 to easily present an interpretation result for the hypothesis to be interpreted. As a result, a user may be allowed to efficiently sort out the hypothesis to be interpreted.

Note that each illustrated constituent element of the information processing device 1 does not necessarily have to be physically configured as illustrated in the drawings. That is, specific forms of distribution and integration of the information processing device 1 are not limited to the illustrated ones, and the whole or a part of the information processing device 1 can be configured by being functionally or physically distributed and integrated in any units according to various loads, use situations, or the like. For example, the hypothesis interpretation unit 11 may be distributed into a functional unit that interprets a hypothesis, a functional unit that aggregates interpretation results, and a functional unit that presents interpretation results. In addition, a storage unit (not illustrated) that stores the hypothesis interpretation-purpose DB 21 and the interpretation rule 22 may be coupled by way of a network, as an external device of the information processing device 1.

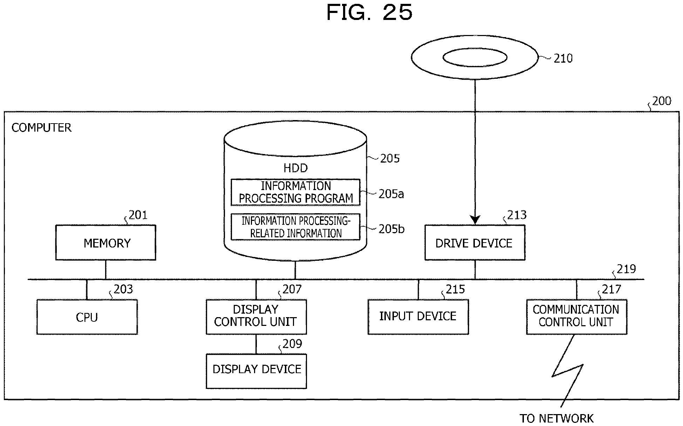

In addition, various processes described in the above embodiments can be implemented by a computer such as a personal computer or a workstation executing a program prepared in advance. Thus, in the following, an example of a computer that executes an information processing program that implements functions similar to the functions of the information processing device 1 illustrated in FIG. 1 will be described. Here, the information processing program that implements functions similar to the functions of the information processing device 1 will be described as an example. FIG. 25 is a diagram illustrating an example of the computer that executes the information processing program.

As illustrated in FIG. 25, a computer 200 includes a central processing unit (CPU) 203 that executes various types of computation processing, an input device 215 that accepts data input from a user, and a display control unit 207 that controls a display device 209. In addition, the computer 200 includes a drive device 213 that reads a program and the like from a storage medium, and a communication control unit 217 that exchanges data with another computer via a network. In addition, the computer 200 includes a memory 201 that temporarily stores various types of information, and a hard disk drive (HDD) 205. Then, the memory 201, the CPU 203, the HDD 205, the display control unit 207, the drive device 213, the input device 215, and the communication control unit 217 are coupled by a bus 219.

The drive device 213 is, for example, a device for a removable disk 210. The HDD 205 stores an information processing program 205a and information processing-related information 205b.

The CPU 203 reads the information processing program 205a to load the read information processing program 205a into the memory 201 and executes the loaded information processing program 205a as a process. Such a process corresponds to each functional unit of the information processing device 1. The information processing-related information 205b corresponds to, for example, the hypothesis interpretation-purpose DB 21, the interpretation rule 22, and the query information 23. Then, for example, the removable disk 210 stores each piece of information such as the information processing program 205a.

Note that the information processing program 205a does not necessarily have to be previously stored in the HDD 205. For example, the program is stored in a "portable physical medium" to be inserted into the computer 200, such as a flexible disk (FD), a compact disk read only memory (CD-ROM), a digital versatile disk (DVD), a magnetooptical disk, or an integrated circuit (IC) card. Then, the computer 200 may read the information processing program 205a from these media to execute the information processing program 205a.

### REFERENCE SIGNS LIST

1 Information processing device
11, 11A Hypothesis interpretation unit
12 DB generation unit
21 Hypothesis interpretation-purpose DB
21a Knowledge table
21b Evidence table
22, 22A Interpretation rule
23 Query information
24 Knowledge data
31 Relationship extraction unit
32 DB integration unit
33 Interpretation rule generation unit
41 Hypothesis interpretation DB
42 Hypothesis display unit

## Claims

1. An information processing program for causing a computer to execute a process comprising:
receiving a hypothesis to be interpreted;
by using a first storage unit that includes, for each piece of knowledge that indicates a plurality of resources and a relationship between the resources, basis information that serves as a basis of the knowledge and a rule identifier connected with a rule used to interpret the hypothesis, acquiring the basis information and the rule identifier that correspond to the hypothesis to be interpreted; and
by using a second storage unit that includes, for each rule identifier, a probability that the rule and the hypothesis coincide with existing knowledge, acquiring the probability of coinciding with the existing knowledge that corresponds to the acquired rule identifier.

2. The information processing program according to claim 1, wherein
the basis information and the probability of coinciding with the existing knowledge that have been acquired are presented for the hypothesis to be interpreted.

3. The information processing program according to claim 1, wherein
a set of the resources is further received,
for the set of the resources, the basis information is acquired from a third storage unit in which the basis information that serves as the basis of the knowledge is connected with each piece of the knowledge that includes a subject, an object, and a predicate that indicates the relationship between the subject and the object, based on the rule included in the second storage unit, and
for the set of the resources, information that includes the relationship between the resources obtained from the rule in the second storage unit, the basis information, and the rule identifier connected with the rule are added to the first storage unit.

4. The information processing program according to claim 3, wherein
the third storage unit that treats a target document as the basis information is generated by using a predetermined relationship extraction process from a natural language, and
a plurality of target databases is integrated by using a predetermined integration process configured to integrate a plurality of existing databases, and the third storage unit that treats the integrated databases as the basis information is generated.

5. The information processing program according to claim 1, wherein
a list of sets of the resources and the relationship to be extracted are further received,
a feature that corresponds to the sets of the resources included in the list and the relationship to be extracted is extracted from a third storage unit in which the basis information that serves as the basis of the knowledge is connected with each piece of the knowledge that includes a subject, an object, and a predicate that indicates the relationship between the subject and the object,
the extracted feature is input, and confidence of the feature is output by applying an explainable artificial intelligence (AI) that treats a variable that indicates presence or absence of the relationship to be extracted, as an objective variable, and
information in which the extracted feature is treated as the rule, which is the information in which the output confidence of the feature is treated as the probability of coinciding with the existing knowledge, and which is the information with which the relationship to be extracted is associated, is added to the second storage unit.

6. The information processing program according to claim 1, wherein
the basis information and the probability of coinciding with the existing knowledge that have been acquired are further stored in a fourth storage unit for the hypothesis to be interpreted, and
the basis information and the probability of coinciding with the existing knowledge for a target hypothesis are extracted and presented from the fourth storage unit, based on a user interface.

7. An information processing program for causing a computer to execute a process comprising:
receiving a hypothesis to be interpreted;
determining whether or not knowledge that corresponds to the hypothesis to be interpreted exists in a third storage unit in which basis information that serves as a basis of the knowledge is connected with each piece of the knowledge that includes a subject, an object, and a predicate that indicates a relationship between the subject and the object, by using a rule that relates to the hypothesis to be interpreted and is included in a second storage unit that includes, for each rule identifier, the rule used to interpret the hypothesis and a probability that the hypothesis coincide with existing knowledge; and
in a case where it is determined that the knowledge that corresponds to the hypothesis to be interpreted exists in the third storage unit, acquiring the basis information that corresponds to the knowledge and the probability of coinciding with the existing knowledge that corresponds to the rule that relates to the hypothesis to be interpreted.

8. An information processing device comprising:
a first acquisition unit that receives a hypothesis to be interpreted, and uses a first storage unit that includes, for each piece of knowledge that indicates a plurality of resources and a relationship between the resources, basis information that serves as a basis of the knowledge, and a rule identifier connected with a rule used to interpret the hypothesis, to acquire the basis information and the rule identifier that correspond to the hypothesis to be interpreted; and
a second acquisition unit that uses a second storage unit that includes, for each rule identifier, a probability that the rule and the hypothesis coincide with existing knowledge, to acquire the probability of coinciding with the existing knowledge that corresponds to the acquired rule identifier.

9. An information processing method implemented by a computer, the information processing method comprising:
receiving a hypothesis to be interpreted;
by using a first storage unit that includes, for each piece of knowledge that indicates a plurality of resources and a relationship between the resources, basis information that serves as a basis of the knowledge and a rule identifier connected with a rule used to interpret the hypothesis, acquiring the basis information and the rule identifier that correspond to the hypothesis to be interpreted; and
by using a second storage unit that includes, for each rule identifier, a probability that the rule and the hypothesis coincide with existing knowledge, acquiring the probability of coinciding with the existing knowledge that corresponds to the acquired rule identifier.
